# EUROPEAN PATENT SPECIFICATION

(11) **EP 3 773 615 B1**
(45) Date of publication and mention of the grant of the patent: **05.07.2023**
(21) Application number: 19723497.4
(22) Date of filing: 09.04.2019
(51) Int. Cl.: A61K 31/728, A61P 1/04, A61P 1/02, A61K 36/886, A61K 35/644, A61K 31/737, A61K 9/00

(54) **LIQUID COMPOSITION FOR USE IN THE TREATMENT OF THE MUCOSA OF THE ORO-PHARYNGO-LARYNGO-ESOPHAGEAL TRACT**
FLÜSSIGE ZUSAMMENSETZUNG ZUR VERWENDUNG BEI DER BEHANDLUNG DER SCHLEIMHAUT DES ORO-PHARYNGO-LARYNGO-ÖSOPHAGEALTRAKTES
COMPOSITION LIQUIDE DESTINÉE À ÊTRE UTILISÉE DANS LE TRAITEMENT DE LA MUQUEUSE DU TRACTUS ORO-PHARYNGO-LARYNGO-OESOPHAGIEN

(30) Priority: 09.04.2018 IT 201800004332
(43) Date of publication of application: 17.02.2021
(73) Proprietor: Sofar Swiss SA, 6900 Lugano (CH)
(72) Inventor: BIFFI, Andrea, 6900 Lugano (CH)
(74) Representative: Benedetto, Marco
(86) International application number: PCT/IB2019/052911
(87) International publication number: WO 2019/197986

(56) References cited:
- WO-A1-2009/138843
- WO-A1-2018/069832
- CN-A- 106 309 306
- CN-A- 107 569 417
- US-A1- 2011 038 945
- TAN S K ET AL: "Comparing the safety, tolerability and efficacy of biotene mouthwash, aloe vera and manuka honey juice for the prophylaxis of oral mucositis", BONE MARROW TRANSPLANTATION, NATURE PUBLISHING GROUP, GB, vol. 41, no. Suppl. 1, 1 March 2008 (2008-03-01), page S402, XP002558096, ISSN: 0268-3369
- PANAHI YUNES ET AL: "Efficacy and safety of Aloe vera syrup for the treatment of gastroesophageal reflux disease: a pilot randomized positive-controlled trial", JOURNAL OF TRADITIONAL CHINESE MADICINE- CHUNG I TSA CHIH YING WEN PAN, vol. 35, no. 6, 15 December 2015 (2015-12-15), pages 632-636, XP029364400, ISSN: 0254-6272, DOI: 10.1016/S0254-6272(15)30151-5

## Description

The present invention relates to an aqueous liquid composition or hydroalcoholic liquid for use in the treatment of the mucosa of the gastro-oesophageal, laryngo-pharyngeal and/or oral tracts, preferably in the treatment of gastroesophageal reflux (GERD). The composition of the present invention comprises a mixture which comprises or, alternatively, consists of a combination of *Aloe vera* gel, hyaluronic acid and honey and, optionally, food and/or pharmaceutical grade additives and/or technological excipients. Said composition being for use in the preventive and/or curative treatment of the symptoms and disorders associated with gastroesophageal reflux and/or laryngopharyngeal reflux (LPR) and the disease caused thereby, for use as an adjuvant in the symptomatic treatment of gastroesophageal and/or laryngopharyngeal reflux disease, or for use in a preventive and/or curative treatment against: (i) lesions of the oral cavity and the pharyngo-laryngo-esophageal tract; (ii) mucositis; and/or (iii) aphthae and/or aphthoid lesions, as defined in the claims.

Gastroesophageal reflux, or GER, is a phenomenon characterised by the temporary upflow of stomach contents into the esophagus, without there necessarily being regurgitation or vomiting. The reflux is principally due to releases of the lower esophageal sphincter (LES), which can be due to insufficient pressure of the LES relative to an increase in abdominal pressure, or a progressive weakening of the closing pressure.

Gastroesophageal reflux disease, abbreviated as GERD (or GORD, Gastro-Oesophageal Reflux Disease), is a chronic disease of gastroenterological interest, which is linked to pathological complications of gastroesophageal reflux (GER) and consists in damage to the mucosa of the esophagus caused by gastric acid.

The most common symptoms of GERD are burning in the gastroesophageal area (retrosternal burning or pyrosis), acidic aftertaste, regurgitation and, more rarely, coughing, painful swallowing (dysphagia and odynophagia), angina-like chest pain, nausea and an increase in salivation (*sialorrhoea*).

In addition to a worsening in the quality of life and a difficulty in taking in food and beverages, GERD can bring about permanent damage to the esophagus, such as chronic inflammation and necrosis of the esophageal epithelium (esophagitis), ulcers, esophageal stenosis, Barrett's esophagus, which is a form of metaplasia characterised by a replacement of the squamous epithelium typical of the esophagus with a cylindrical epithelium, and tumours. Subjects with persistent symptoms of GERD are considered to be at high risk of developing adenocarcinoma of the distal tract of the esophagus.

An assessment of the damage to the esophageal mucosa is generally performed by means of endoscopic examinations, e.g. gastroscopy or pH-metry.

Therapy of GERD is currently based on some basic hygienic and dietetic rules (cessation of the use of tobacco and intake of alcoholic beverages) and the administration (for more or less extended periods) of drugs belonging to the classes of proton pump inhibitors (PPIs), which considerably inhibit the production of acid in the stomach and have replaced antihistamine (anti-H2) drugs. In order to obtain relief from the symptoms, antacids and alginate-based drugs can also be used.

However, the administration of these drugs can be ineffective in some subjects and can have side effects at the intestinal level (diarrhoea, flatulence, abdominal pain), skin eruptions, heart palpitations and an increase in osteoporosis and bone fragility in the case of long-term treatments.

Laryngopharyngeal reflux (LPR) is a little known pathology that is difficult to diagnose and if treated it does not cause significant problems, but without adequate treatment it can prove to be a serious pathology, dangerous for the airways.

Laryngopharyngeal reflux consists in a retrograde reflux of gastric juices which rise up from the stomach through the esophagus and arrive at the pharynx and larynx, the first upper airways. At this level, due to the effect of gastric juices, the mucosa is damaged and thus become inflamed.

This pathology is caused by a dysfunction of the upper esophageal sphincter, i.e. a group of muscles situated in the initial part of the esophagus which normally has the function of closing and opening the esophagus, by contracting and dilating, and preventing the esophageal content from rising up through the airways and air from going into the stomach.

Unlike what happens in the better known gastroesophageal reflux, the gastric juices and gases that rise from the stomach pass through the esophagus rapidly, so much so that those who suffer from laryngopharyngeal reflux do not complain of ailments such as burning or acidity and in these cases one refers to "silent reflux". In the majority of cases laryngopharyngeal reflux manifests itself with symptoms that are unlikely to be associated with reflux and make it difficult for the physician to diagnose. The typical symptoms are the ones of many common disorders of the oral cavity:
chronic sore throat, hoarseness, frequent need to clear one's throat, chronic dry cough (lasting more than three weeks), night time coughing, regurgitation due to position and night-time regurgitation, dysphonia, thick, abundant mucus, sensation of something stuck in the throat, the so-called "pharyngeal bolus", and in some cases also retrosternal burning.

The symptoms are due to the direct damage that the gastric juices and gases coming from the stomach cause to the outermost tissues of the pharynx and larynx.

This is why, in addition to the drugs against gastric acidity traditionally used to treat laryngopharyngeal reflux, medicinal specialties or medical devices based on hyaluronic acid, for example, in the form of meltin-mouth tablets, are available today.

However, the administration of these products can be ineffective in some subjects and can cause side effects similar to the ones caused by drugs against gastric acidity.

Finally, there are some known liquid compositions present in the market containing chondroitin sulphate sodium, sodium hyaluronate and bioadhesive polymers, such as, for example, the ones described in EP 2296670 B1 - (see, for example, examples 1-6), which are not devoid of limits, for example in terms of effectiveness.

The technical problem of providing a valid solution for the treatment or the prevention of the symptoms associated with gastroesophageal reflux, and of GERD, and/or laryngopharyngeal reflux which overcomes the drawbacks of the prior art, in particular with regard to the side effects, has not been solved to date. More in general, the technical problem of providing a valid solution for the treatment of (i) lesions of the oral cavity and the pharyngo-laryngo-esophageal tract, (ii) mucositis, and/or (iii) aphthae and/or aphthoid lesions which are free of side effects, has not been solved to date.

Furthermore, the problem of providing a valid solution that is both effective and capable of performing an anti-inflammatory action and promotes the process of tissue re-epithelisation and cicatrisation against lesions caused to the mucosa of the oro-laryngo-pharyngo-esophageal tract as a result of the upflow of acidic and/or basic vapours from the stomach towards the upper parts of the digestive system, effectively and rapidly, remains unsolved.

In order to overcome said technical problems, the present invention provides a composition, comprising substances of natural origin, which is capable of effectively and rapidly treating (preventing and/or curing) the symptoms associated with gastroesophageal reflux and/or laryngopharyngeal reflux, and of the disease caused thereby, in particular which is capable of performing an anti-inflammatory action and promoting the process of tissue re-epithelisation and cicatrisation against lesions caused to the mucosa of the oro-laryngo-pharyngo-esophageal tract. Furthermore, the present invention provides a composition that is free of the side effects present in prior art treatments, but is easy to prepare and economically advantageous.

Description of the figures:
Figure 1: it shows the percentage of caffeine over time under conditions of pH=7 in a model of reconstituted human esophageal epithelium treated with the compositions undergoing study.
Figure 2: it shows the percentage of caffeine over time under acidic conditions (pH=3.3) in a model of reconstituted human esophageal epithelium treated with the compositions undergoing study.
Figure 3: it relates to the histological analysis on reconstituted human oesophageal epithelium (HOE2E/S/5), damaged area treated with the composition according to the invention (B).
Figure 4: it relates to the histological analysis on HOE2E/S/5, undamaged area treated with a comparative composition (B).
Figure 5: it relates to the histological analysis on HOE2E/S/5, damaged area treated with the composition according to the invention (A).
Figure 6: it relates to the histological analysis on HOE2E/S/5, undamaged area treated with a comparative composition (A).
Figure 7: it relates to the histological analysis on HOE2E/S/5, damaged area treated with an *Aloe vera:* honey gel mixture (C).
Figure 8: it relates to the histological analysis on HOE2E/S/5, undamaged area treated with an *Aloe vera:* honey gel mixture (C).
Figure 9: it shows the percentage of caffeine over time under conditions of pH=7 in a model of reconstituted human esophageal epithelium treated with the compositions undergoing study.
Figure 10: it shows the percentage of caffeine penetration over time under acidic conditions in a model of reconstituted human esophageal epithelium treated with the compositions undergoing study.

The present invention relates to an aqueous liquid composition or hydroalcoholic liquid (C) comprising a mixture which comprises or, alternatively, consists of a combination of the following substances:
(a) an *Aloe vera* gel (as defined in the context of the present invention); and
(b) a hyaluronic acid or a salt thereof; and
(c) a honey, provided that said honey is not honey with non-peroxide antibacterial activity and provided that said honey is not clover honey filtered and processed at a temperature between 100 and 140⁰ F; and, optionally, at least one excipient, or additive, suitable for pharmaceutical or food use.

Honey with non-peroxide antibacterial activity means a substance that has an antibacterial activity demonstrable in situ that is not destroyed by the enzyme catalase and whose antibacterial activity does not depend on the production or presence of hydrogen peroxide, as described for example in patent application US20110038945 A1, see, by way of example, paragraph [0142]. Examples of types and sources of honey with a substantial non-peroxide antibacterial activity are the ones described, for example, in patent application US20110038945; see, by way of example, paragraphs [0229]-[0237].

An example of clover honey filtered and processed at a temperature between 100 and 140⁰ F (degrees Fahrenheit) is clover honey from Montana or North Dakota described, for example, in patent application US2009/0208588 A1. The processes of filtration and pasteurisation of said honey are the ones known to the person skilled in the art.

In a first embodiment, the composition (C) is an aqueous liquid solution for use in a method of preventive and/or curative treatment, of laryngopharyngeal reflux (RLF) disease, or for use as an adjuvant in a method for the symptomatic treatment of laryngopharyngeal reflux (RLF)disease.

In a second embodiment, the composition (C) is a hydroalcoholic liquid solution. Advantageously, said hydroalcoholic liquid composition comprises at least one alcohol in an amount by volume comprised from 0.01 to 3%, preferably comprised from 0.05 to 2.5%, more preferably from 0.1 to 1.0%, wherein said amounts are relative to the total volume of the composition (C).

Advantageously, said at least one alcohol is ethyl alcohol or any other alcohol suitable for forming a hydroalcoholic solution that may be administered to human or animal subjects in addition to being compatible with the components (a)-(c) and, optionally, (d).

In the context of the present invention the term liquid composition (C) or composition (C) according to the present invention includes the aqueous liquid composition or, alternatively, the hydroalcoholic liquid composition.

The present invention relates to the aqueous liquid composition or hydroalcoholic liquid (C) of the present invention, comprising (a), (b), (c) and, optionally, (d) as defined in the context of the present invention, for use in the treatment of the gastroesophageal reflux and/or laryngopharyngeal reflux; for use as an adjuvant in the symptomatic treatment of gastroesophageal reflux and/or laryngopharyngeal reflux disease; or for use in a preventive and/or curative treatment against: (i) lesions of the oral cavity (e.g. tongue and palate) and of the pharyngo-laryngo-esophageal tract; (ii) mucositis; and/or (iii) aphthae and/or aphthoid lesions, as defined in the claims.

It has been found that the administration of the composition (C) according to the present invention is capable of considerably decreasing/reducing and/or eliminating the symptoms and disorders associated with gastroesophageal reflux and laryngopharyngeal reflux, thus alleviating the effects of the disease itself. Advantageously, the liquid composition (C) of the present invention acts along the esophagus and the laryngopharyngeal tract and performs an anti-inflammatory action, promotes the process of tissue re-epithelisation and a cicatrising action against lesions caused to the mucosa as a result of the upflow, from the stomach towards the esophagus and the laryngopharyngeal tract, of vapours and acidic substances or substances of an acidic character and/or of basic substances or substances of a basic character and/or, alternatively, a mix of acid and basic substances. In particular, the liquid composition (C) of the present invention enables better contact of the individual components/substances contained in the mixture with the oro-pharyngo-laryngo-esophageal wall and favours the protection, lubrication and repair thereof.

The composition (C) according to the present invention (aqueous or hydroalcoholic) can be in the form of a clear solution (without sediment) or a suspension (i.e. a liquid phase that exhibits a visible opalescence, a solid suspended in a liquid or semi-liquid mass or a sediment that can be suspended by shaking) and it can be a dense, viscous liquid (like caramel) or a fluid, flowable one (like water) or it can be a two-phase liquid/liquid system. The composition (C) according to the present invention is preferably not an emulsion.

In a preferred embodiment, the composition of the present invention is in the form of a syrup, for example having a specific weight of about 1.2-1.3 kg/dm3 at 20°C and a viscosity of about 200-205 mPa.s at 20°C, comprising water and at least one food or pharmaceutical grade ingredient or additive and/or technological excipients.

In the context of the present invention, "treatment" means an intervention comprising the administration of a substance, or mixture of substances or combination thereof, having the aim of eliminating, reducing/decreasing or preventing a pathology or disease and the symptoms or disorders thereof.

Unless specified otherwise, the content of a component or substance in a composition refers to the percentage by weight of that component or substance relative to the total weight of the composition. Unless specified otherwise, the indication that a composition "comprises" one or more components or substances means that other components or substances can be present in addition to the one or ones specifically indicated.

The composition of the present invention is to be understood as for either human or veterinary use, i.e. as a preparation to be applied to animals with the uses and methods known to the person skilled in the art.

In the context of the present invention, and in accordance with its common meaning, the term "honey" refers to the sweet natural product produced by bees (e.g. *Apis mellifera*) from the nectar of one or more plant varieties of any type or from secretions originating from living parts of plants or substances secreted by sucking insects that are found on living parts of plants, which they collect, transform, by combining them with specific substances of their own, deposit, dehydrate, store and leave in honeycombs to ripen and mature, according to the definition in Italian Legislative Decree no. 179 of 21 May 2004, transposing Directive 2001/110/EC relating to the production and marketing of honey. Said honey can be obtained through the standard processes known to the person skilled in the art (for example comprising extraction, separation, decanting, filtration, guided crystallisation and similar operations). In the context of the present invention, the term "honey" also comprises the products that can be obtained from natural honey, including those for industrial use, for example through refining processes or heat treatments, such as pasteurisation. In the context of the present invention, the term "honey" means what is described above, provided that said honey is not honey with a non-peroxide antibacterial activity (as defined above) and provided that said honey is not clover honey filtered and processed at a temperature between 100 and 140° F (as defined above).

The use of honey having a peroxide activity greater than 5 micrograms of hydrogen peroxide per gram of honey, in combination with raw food fibres (such as wheat bran) for the production of a composition for combating disorders such as gastroesophageal reflux is known.

By way of non-limiting example, the honey usable in the composition of the present invention can have a pH between 3.5 and 4.5, a weight loss on drying of 18% (by weight relative to the total weight of the honey) and a content of reducing sugars in the dry product of 70% (by weight relative to the total weight of the honey).

In the context of the present invention, *"Aloe vera* gel" refers to the generally colourless mucilaginous gel obtained from the parenchymatous tissue of the leaves of *Aloe vera* (L) Burm. f. or *Aloe barbadensis* Mill. *Aloe vera* gel does not have a use as a food substance or fibre and must not be confused with the juice (*Aloe vera* juice), which is obtained from the same plant by incision and drying. In the monograph on *Aloe vera* gel in *"*WHO monographs on selected medicinal plants" (Vol. 1 World Health Organization, Geneva, 1999 pp. 43-49) it is stated that its principal components, besides water, are polysaccharides (pectins, hemicelluloses, glucomannans, acemannans and mannose derivatives) and that administration of *Aloe vera* gel has not been shown to have any significant therapeutic effect (pg. 45).

Furthermore, the term *"Aloe vera* gel" in the context of the present invention must not be confused with the general term *"Aloe vera",* since *Aloe vera* gel is a specific part obtained from the plant of *Aloe vera,* namely, the mucilaginous gel obtained from the parenchymatic tissue of leaves of *Aloe vera* (L) Burm. f. or *Aloe barbadensis* Miller.

The present inventors have found, by contrast, that oral administration of *Aloe vera* gel, in combination with honey, provided that said honey is not honey with a non-peroxide antibacterial activity (as defined above) and provided that said honey is not clover honey filtered and processed at a temperature between 100 and 140⁰ F (as defined above), and hyaluronic acid, in accordance with the present invention, makes it possible to considerably decrease or completely eliminate, effectively and rapidly, the symptoms and the disorders associated with gastroesophageal reflux and laryngopharyngeal reflux and enables treatment of (i) lesions of the oral cavity (e.g. tongue and palate) and of the pharyngo-laryngo-esophageal tract; (ii) mucositis; and/or (iii) aphthae and/or aphthoid lesions.

In the composition (C) of the present invention, the *Aloe vera* gel is preferably a lyophilised inner leaf gel, more preferably from *Aloe barbadensis* Miller.

By way of non-limiting example, the *Aloe vera* gel used in the present invention can have a pH between 3 and 6, preferably between 3.5 and 5.5 or 3.7 and 4.2, and have a content of aloin lower than 1 ppm.

The *Aloe vera* gel of the present invention is obtained by means of standard methods of preparation. One example of a method of preparation is the following: the aloe gel is obtained by means of the following steps: selection of leaves of adult aloe plants; removal of the outer part, where the thorns and the glands secreting anthraquinones (these substances are particularly known for their laxative action) are concentrated; pressing of the aloe pulp, from which the gel is obtained, leaving behind the fibrous residues; stabilisation of the gel with specific additives to protect the active ingredients from chemical oxidation; purification of the gel; packaging in dark or opaque hermetic containers, since aloe gel degrades easily if exposed to oxygen and direct light; storage in a cool place (4-20°C), since aloe gel is strongly compromised by high temperatures elevate. Alternatively, the *Aloe vera* gel can be prepared as a lyophilised *Aloe vera* inner leaf gel according to the methods known to the person skilled in the art.

Hyaluronic acid is a non-sulphated glycosaminoglycan having an unbranched polysaccharide chain deriving from the condensation of disaccharide units which are formed, in turn, from residues of glucuronic acid and N-acetylglucosamine, linked together by alternating glycosidic bonds β1→4 and β1→3 (CAS number 9004-61-9).

Hyaluronic acid is widely used also in the form of a salt, for example as a sodium salt, via injections, in aesthetic surgery and dermatology, in otologic surgery, in ophthalmic surgery and in arthrology. Moreover, hyaluronic acid is widely used for topical application against inflammations or ulcerous lesions of the mouth and as a filler for skin applications in facial and body care products.

In the context of the present invention, the hyaluronic acid can be in the acid or salt form, for example as a sodium salt; the hyaluronic acid is preferably in linear form.

The mixture contained in the composition (C) according to the present invention may comprise hyaluronic acid, or salts thereof, having a different origin and various intervals of molecular weights.

The hyaluronic acid, or salts thereof, used in the mixture contained in the composition (C) of the present invention, in combination with the substances (a) and (c) and, optionally, (d), is linear or branched; the hyaluronic acid is preferably in linear form.

The mixture contained in said composition (C) according to the present invention, comprising (a), (b), (c) and, optionally, (d), preferably comprises linear or branched hyaluronic acid, or salts thereof, having a molecular weight comprised from 400 to 900 kDa, preferably from 600 to 800 kDa; the hyaluronic acid is preferably in linear form.

More preferably, the mixture contained in said composition (C) according to the present invention comprises sodium hyaluronate having a molecular weight comprised from 600 to 800 kDalton (CAS No. 9067-32-7).

In a preferred embodiment, the composition (C) of the present invention, comprising (a), (b), (c), further comprises at least one other glycosaminoglycan (GAG), or a salt thereof, in addition to the hyaluronic acid; more preferably, said GAG is a salt of chondroitin; even more preferably, said GAG is the substance (d) chondroitin sulphate.

The chains of chondroitin, or of the derivatives thereof such as sulphate, are branchless polysaccharides of variable length containing two alternating monosaccharides: D-glucuronic acid (GlcA) and N-acetyl-D-galactosamine (GaINAc), wherein the residues of GlcA can be epimerised into L-iduronic acid (the resulting disaccharide is called Dermatan sulphate).

It is known from the Merck Index (14-th edition) that chondroitin sulphate is a generic term that indicates a polymer having an average molecular weight of about 50,000 Da.

There exist various forms of chondroitin sulphate, such as, for example, chondroitin 4-sulphate A [CAS No. 24967-93-9], the sodium salt thereof [CAS No. 9082-07-9] or the disodium salt thereof [CAS No. 39455-18-0]. The form chondroitin 6-sulphate (chondroitin sulphate C) also exists-CAS No. 25322-46-7.

In the context of the present invention, it is envisaged to use one of the above-mentioned forms of chondroitin sulphate (d) in combination with the substances (a), (b) and (c) and said chondroitin (d) is chicken chondroitin.

Preferably, the chondroitin sulphate (optionally in the form of a sodium salt) in the composition for use according to the present invention has an average molecular weight that depends on the specific form of chondroitin used.

It has been found that the administration of the liquid composition (C) according to the invention, also comprising chondroitin sulphate, has the effect of further reducing the symptoms associated with gastroesophageal and/or laryngopharyngeal reflux, practically in the absence of undesirable effects.

The chondroitin sulphate in the composition (C) of the invention preferably derives from chicken, fish (e.g. shark chondroitin sulphate, CAS number 9082-07-9), bovines or swine or is of vegetable origin; more preferably, the chondroitin sulphate, or salts thereof, used in the composition of the present invention derives from chicken. By way of non-limiting example, the chondroitin sulphate in the composition according to the present invention may be chicken chondroitin sulphate sodium salt and contain, relative to the total weight of the composition, at least 91.5% by weight and no more than 8.5% by weight of chondroitin sulphate sodium salt; furthermore, it may have a protein content no greater than 6% by weight and have a pH comprised from 5.5 to 7.5 and/or a specific rotation comprised from 10° to 20° (for example determined with the methods of the European Pharmacopoeia 7.0).

In a preferred embodiment, the liquid composition (C) according to the present invention comprises:
- (a) an *Aloe vera* gel in an amount comprised from 0.01 to 5%, preferably comprised from 0.1 to 0.5%;
- (b) a hyaluronic acid in an amount comprised from 0.01 to 5%, preferably comprised from 0.1 to 0.5%;
- (c) a honey in an amount comprised from 5 to 50%, preferably comprised from 10 to 40%; and
- (d) a salt of chondroitin sulphate, preferably a sodium salt, if present, in an amount comprised from 0.1 to 10%, preferably comprised from 1 to 4%; wherein all the amounts are by weight relative to the total weight of (C).

In a preferred embodiment, the liquid composition (C) of the present invention comprises, per 100 ml of (C);
- (a) an *Aloe vera* gel in an amount comprised from 100 to 500 mg, preferably comprised from 150 to 300 mg;
- (b) a hyaluronic acid in an amount comprised from 100 to 500 mg, preferably comprised from 100 to 300 mg;
- (c) a honey in an amount comprised from 10 to 50 g, preferably comprised from 20 to 30 g; and, optionally;
- (d) a salt of chondroitin sulphate in an amount comprised from 1 to 5 g, preferably comprised from 2 to 3 g.

In a more preferred embodiment, the liquid composition (C) comprises, per 100 ml of (C):
- (a) lyophilised *Aloe vera* gel 0.25 g;
- (b) hyaluronic acid 0.2 g;
- (c) honey 25 g.

In another more preferred embodiment, the liquid composition (C) comprises, per 100 ml of (C):
- (a) lyophilised *Aloe vera* gel: 0.25 g;
- (b) hyaluronic acid: 0.2 g;
- (c) honey: 25 g;
- (d) a salt of chondroitin, preferably chondroitin sulphate: 2.5 g.

The liquid composition (C) according to the present invention, in addition to the components (a)-(c) and, optionally, (d), can further comprise other active ingredients such as, by way of non-limiting example, anti-inflammatory agents, oral cavity disinfectants, antacids, products for the treatment of gastroesophageal reflux and/or laryngopharyngeal reflux (e.g. prokinetics, alginates) and mixtures thereof.

The composition (C) as defined above, comprising (a), (b), (c) and, optionally, (d), can further comprise at least one excipient, or additive, i.e. a substance devoid of therapeutic activity, suitable for pharmaceutical or food use.

In the context of the present invention the acceptable ingredients for pharmaceutical or food use comprise all the auxiliary substances known to the person skilled in the art and suitable for the preparation of liquid forms for oral administration, such as, by way of non-limiting example, diluents, solvents (including water, glycerine, ethyl alcohol), solubilisers, thickeners, sweeteners, flavourings, colourants, lubricants, surfactants, antimicrobials, antioxidants, preservatives, pH stabilising buffers and mixtures thereof. Non-limiting examples of such substances are maltodextrins, phosphate buffers, bases such as sodium hydroxide, xanthan gum, guar gum, fructose, and natural or artificial flavourings.

In the context of the present invention, composition(s) is meant to include pharmaceutical compositions, compositions for medical devices and compositions for dietary supplements.

In a preferred aspect, the composition (C) of the present invention is a pharmaceutical composition, a composition for a medical device or a composition for a dietary supplement.

In the context of the present invention, the term "medical device" is used with the meaning according to Italian Legislative Decree no. 46 of 24 February 1997, i.e. it indicates a substance or another product, whether used alone or in combination, intended by the manufacturer to be used for human beings for the purpose of diagnosis, prevention, monitoring, treatment or alleviation of a disease, and which does not achieve its principal intended action in or on the human body by pharmacological, immunological or metabolic means, but which may be assisted in its function by such means.

By way of non-limiting example, the composition (C) of the present invention, for example in the form of a medical device or drug or dietary supplement, can be in a pharmaceutical form of a syrup, liquid or semisolid preparation, gel, suspension, solution, two-phase liquid system and equivalent forms. It remains understood, however, that the composition of the present invention is not in the form of an oil-water microemulsion.

The composition (C) of the present invention is preferably in the form of a water-based syrup.

The present invention relates to a composition comprising a mixture which comprises or, alternatively, consists of:
(a) an *Aloe vera* gel;
(b) a hyaluronic acid; and
(c) a honey, provided that said honey is not honey with a non-peroxide antibacterial activity and provided that said honey is not a clover honey filtered and processed at a temperature between 100 and 140°F; and, optionally,
(d) chondroitin sulphate or a salt thereof, preferably a sodium salt; and, optionally,
pharmaceutical and/or food grade additives and/or excipients; said composition being for use in the treatment and/or in the prevention of gastroesophageal reflux and/or laryngopharyngeal reflux disease, for use as an adjuvant in the symptomatic treatment of gastroesophageal reflux and/or laryngopharyngeal reflux disease; or for use in a preventive and/or curative treatment against: (i) lesions of the oral cavity (e.g. tongue and palate) and of the pharyngo-laryngo-esophageal tract; (ii) mucositis; and/or (iii) aphthae and/or aphthoid lesions, as defined in the claims.

For the sake of clarity, in order to achieve the object of the present invention, (a), (b), (c) and, optionally, (d) can be also administered separately and in any order but, preferably, (a), (b), (c) and, optionally, (d) are administered to a subject simultaneously, and even more preferably in a single composition so as to obtain a more rapid effect and facilitate administration.

When (a), (b), (c) and, optionally, (d) are administered in a single composition, said single composition corresponds to the composition (C) in liquid, preferably aqueous, form of the present invention.

Said composition according to the present invention preferably further comprises a salt of chondroitin, preferably of chondroitin sulphate, such as a sodium salt of chondroitin sulphate. The source of the chondroitin is preferably chicken.

The following examples are provided in order to illustrate some embodiments of the invention, without any intention of limiting the scope thereof.

In the following examples, the component (c) honey is as defined in the present invention, i.e. honey provided that said honey is not honey with a non-peroxide antibacterial activity and provided that said honey is not clover honey filtered and processed at a temperature between 100° and 140⁰ F as defined above.

### EXAMPLE 1

A composition (C) according to the invention was prepared in the form of a syrup (total volume of 100 ml) comprising the following ingredients:

| | | |
|---|---|---|
| - | Lyophilised *Aloe vera* gel (a): | 0.25 g |
| - | Sodium hyaluronate (b): | 0.23 g |
| - | Honey (c): | 25 g |
| - | Fructose: | 23.8 g |
| - | Hydroxypropyl methylcellulose: | 3 g |
| - | Benzoates (preservatives): | 0.15 g |
| - | Flavourings: | 0.3 g |
| - | Thickener: | 0.1 g |
| - | Purified water: | quantum sufficit (q.s.) to 100ml |

### EXAMPLE 2

A composition (C) according to the invention was prepared in the form of a syrup (total volume of 100 ml) comprising the following ingredients:

| | | |
|---|---|---|
| - | Lyophilised *Aloe vera* gel (a): | 0.25 g |
| - | Sodium hyaluronate (b): | 0.23 g |
| - | Honey (c): | 25 g |
| - | Chondroitin sulphate sodium (d): | 2.8 g |
| - | Fructose: | 23.8 g |
| - | Hydroxypropyl methylcellulose: | 3 g |
| - | Benzoates (preservatives): | 0.15 g |
| - | Flavourings: | 0.3 g |
| - | Thickener: | 0.1 g |
| - | Water: | q.s. to 100 ml |

### EXAMPLE 3

A composition (C) according to the invention was prepared in the form of a syrup (total weight 100 g) comprising the following ingredients:

| | | |
|---|---|---|
| - | Lyophilised *Aloe vera* gel (a): | 0.21 g |
| - | Sodium hyaluronate (b): | 0.24 g |
| - | Honey (c): | 21 g |
| - | Chondroitin sulphate sodium (d): | 2.3 g |
| - | Fructose: | 6.2 g |
| - | Potassium/dipotassium phosphate buffer | 2.1 g |
| - | Sodium hydroxide | 0.15 g |
| - | Benzoates (preservatives): | 0.15 g |
| - | Xanthan gum | 0.25 g |
| - | Flavourings: | 0.3 g |
| - | Maltodextrins | 11.6 g |
| - | Thickener (guar flour) | 0.2 g |
| - | Water: | 55 g |
| - | Other excipients: | q.s. to 100 g |

In a non-claimed aspect of the present disclosure, the compositions of examples 1, 2 and 3 were orally administered to subjects affected by gastroesophageal reflux disease (GERD), diagnosed through endoscopic analysis.

All subjects reported an improvement in the symptoms associated with GERD, while no significant side effects were caused.

In the formulation in question (liquid composition (C), preferably aqueous), the aforesaid invention lends itself to being used in subjects of all ages, from newborns to the elderly.

The liquid composition (C) of the present invention (which comprises a mixture which comprises or, alternatively, consists of (a), (b), (c) and, optionally, (d)): is preferably an association (mixture) of Chondroitin Sulphate, Hyaluronic acid, *Aloe vera* gel and Honey, endowed with the following properties:
- Hydrating, moistening, lubricating, viscosity enhancing, film-forming and protective, with a barrier effect due to the formation of a viscous layer that coats the esophageal mucosa by adhering to it ("gripping" action), thus exerting a barrier action useful for preventing the contact of the mucosa itself with external agents, and contributing to alleviating irritative states and favouring, therefore, a proper trophism and correct functionality. By virtue of such effects, the aforesaid invention is capable of exerting a trophic, anti-inflammatory, analgesic (pain-killing), reparative (cicatrising), restorative and re-epithelising (it favours tissue regeneration) action;
- Emollient and soothing effect, preferably on the epithelium and the oro-pharyngo-laryngeal and esophageal mucosa, useful for providing relief of the most common symptoms of GERD and/or of laryngopharyngeal reflux and calming coughs and other related symptoms. By virtue of such effects, the aforesaid invention is capable of exerting a refreshing, balsamic, calming and cough-sedating action;
- It protects the tissue of the damaged gastroesophageal and laryngopharyngeal tract and favours the regeneration of the damaged mucosa, and is useful for preventing the damage induced by the irritating action due, in particular, to contact with the substances regurgitated in the gastroesophageal tract. By virtue of such effects, the aforesaid invention is capable of exerting a gastroprotective action.

The liquid composition (C) comprising a mixture which comprises or, alternatively, consists of (a), (b), (c) and, optionally, (d) is in the form of solution or syrup or soluble sachets and enables better contact of the individual components/substances with the oro-pharyngo-laryngo-esophageal wall, favouring the protection, lubrication and repair thereof:
- Chondroitin-Sulphate adheres to the gastroesophageal mucosa and protects it by effectively isolating it from the attack of gastric juices;
- Hyaluronic acid, in combination with the adhesive properties of chondroitin sulphate, protects the damaged gastric tissue and favours its regeneration;
- Honey exerts a soothing, hydrating and protective action due to the formation of a film that coats the esophageal mucosa, with a barrier action, protecting the irritated mucosa and hydrating it, thus alleviating the sensation of pain. Furthermore, honey, by acting at the level of the oropharyngeal mucosa, alleviates the throat irritations provoked by the acidity of the reflux;
- *Aloe vera* gel, together with the properties of the honey, exerts a refreshing, soothing and protective action on the esophageal mucosa, providing a barrier effect. In fact, *Aloe vera,* thanks to its content of mucopolysaccharides, possesses gastroprotective properties, because by distributing themselves over mucosa of the stomach, they form a sort of film that protects the entire gastric tract from acids or irritant agents that would alter its correct functioning.

Preferred intended uses of the aforesaid liquid composition (C), comprising a mixture which comprises or, alternatively, consists of (a), (b), (c) and, optionally, (d), are: for use as an adjuvant in the symptomatic treatment of gastroesophageal reflux and/or laryngopharyngeal reflux disease; or for use in a preventive and/or curative action against: (i) lesions of the oral cavity (e.g. tongue and palate) and of the pharyngeallaryngeal-esophageal tract; (ii) mucositis; (iii) aphthae and/or aphthoid lesions.

The characteristics of the liquid composition (C) according to the invention are revealed by the experimental results presented below. In particular, the liquid composition (C) according to the invention exhibits a better film-forming/mucoadhesive/protective action compared to compositions present on the market and an *Aloe vera* gel and honey mixture, as demonstrated in the *in vitro* test described below (test I). At the same time, the liquid composition (C) according to the invention has a neutral impact on the integrity of the barrier. Furthermore, the liquid composition (C) according to the invention effectively exerts a re-epithelising/reparative action by promoting the repopulation of the injured area and favouring the late phase of tissue re-epithelialisation (proliferative phase following the migratory phase) over a long period of treatment as demonstrated in the *in vitro* test described below (test II).

### EXPERIMENTAL TRIALS (a)

### In vitro test

The model used was reconstituted human oesophageal epithelium (HOE2E/S/5), produced by Episkin ^{®}, Lyons (F).

### a.I) Film-forming/mucoadhesive/protective action

### a.I-i) Compositions undergoing study and Controls

- comparative COMPOSITION (A),
- COMPOSITION according to the invention (B),
- comparative ALOE:HONEY MIXTURE (C),
- negative control: saline solution (NC),
- positive control: petrolatum (V).

The comparative COMPOSITION (A) (Esoxx^{®} one) is a prior art composition comprising sodium hyaluronate, chondroitin sulphate sodium and bioadhesive polymers such as poloxamers or copolymers of ethylene and propylene oxide known as Lutrol^{®}, for example Lutrol F127, and/or polyvinylpyrrolidone, and other components according to what is specified in Table 1. In particular, the comparative composition (A) corresponds to the one described in Example 1 of patent EP 2296670 B1.

**Table 1**

| | | |
|---|---|---|
| DEIONISED WATER | 74.015 | 8882 mg |
| SODIUM HYALURONATE | 1% | 120 mg |
| CHONDROITIN SULPHATE SODIUM | 2.5% | 300 mg |
| XYLITOL C | 18% | 2160 mg |
| LUTROL F127 | 2% | 240 mg |
| POLYVINYLPYRRHOLIDONE Kw 24-32 | 2% | 240 mg |
| SODIUM BENZOATE | 0.067% | 8 mg |
| POTASSIUM SORBATE | 0.2% | 24 mg |
| FLAVOURING + COLOURANT | 0.22% | 26 mg |

The liquid composition according to the invention (B) (GERDOFF PROTECTION^{®}) is a composition according to what is specified in Table 2. The composition has a pH value comprised from 6.5 to 7.5 and a density value comprised from 1.19 to 1.21 g/ml (measured at 25°C with a pycnometer in the collection container).

**Table 2**

| **INGREDIENTS (GERDOFF PROTECTION^{®})** | **%** | **Amt. per dose** |
|---|---|---|
| Purified water | 55.307 | 6636.855 mg |
| Honey | 21.090 | 2530.800 mg |
| Maltodextrins | 11.577 | 1389.240 mg |
| Fructose | 6.221 | 746.520 mg |
| Chondroitin sulphate sodium | 2.315 | 277.778 mg |
| Dipotassium phosphate | 1.400 | 168.000 mg |
| Potassium phosphate | 0.700 | 84.000 mg |
| Xanthan gum | 0.250 | 30.000 mg |
| Sodium hyaluronate | 0.231 | 27.778 mg |
| Lyophilised *Aloe vera* gel (*Aloe barbadensis* Miller) | 0.208 | 25.000 mg |
| *Cream caramel* flavouring | 0.200 | 24.000 mg |
| Guar seed (*Cyamopsis tetragonoloba L. Taub*.) flour | 0.200 | 24.000 mg |
| Sodium hydroxide | 0.150 | 18.030 mg |
| Sodium methyl paraben | 0.120 | 14.400 mg |
| Propyl 4-hydroxybenzoate sodium salt | 0.030 | 3.600 mg |
| Total | 100.0000% | 12000.0000 mg |

The ALOE:HONEY MIXTURE (C) is a lyophilised *Aloe vera* gel (*Aloe barbadensis* Miller):honey mixture in a 100:1 ratio that has the following composition per volume of 100 ml:

| | |
|---|---|
| • purified water | 78.79% |
| • honey | 21.00% |
| • *Aloe vera* gel | 0.21% |

The honey present both in the Composition (B) and in the Mixture (C) is not a honey with a non-peroxide antibacterial activity, much less a clover honey filtered and processed at a temperature between 100 and 140⁰ F.

### a-I-ii) Study design and Results

The aforesaid compositions A, B and C (point a-I-i) were tested for their film-forming properties on a reconstituted human esophageal epithelium (HO2E/S/5) as a biological model under different pH conditions: standard = pH 7.0 and acid conditions = pH 3.3.

In terms of morphology (multilayer or epithelium) and biochemical and physiological properties, reconstituted human epithelium models *in vitro* are close to human tissues in vivo and today represent the most promising alternative to animals (ex vivo) for evaluating the topical products applied (Gordon et al., 2015, Zuang V. 2016).

In particular, the following were evaluated:
the kinetics of caffeine penetration (film-forming effectiveness) induced by the products A, B and C and they were compared with the negative control (NC) and the positive control (V).

### PROTOCOL

0.5 cm² of reconstituted human oesophageal epithelium (HOE2E/S/5) was used. HO2E/S/5 was removed from the nutrient agar solution under a sterile laminar air flow cabin. The inserts were transferred onto a 6-well plate previously filled with maintenance medium (1 ml / well) at room temperature and incubated at 37° C, 5% CO₂, saturated humidity overnight.

The following day, the maintenance medium was changed and the HO2E/S/5 was pre-wet with 15µL of saline solution. After 15 minutes 30 µl of NC, V, A, B or C were applied for 15 minutes at room temperature. After 15 minutes of application, 100 µl of a 0.5% caffeine solution (CAFFEINE 1 mg / cm²) as a standard solution at pH=7 or an acid solution at pH=3.3 were applied topically, directly and evenly on the epithelium for 2 hours at room temperature.

Kinetics of caffeine penetration (film-forming effectiveness):
15 minutes, 1 hour and 2 hours after application, the fluid of the receptor (saline solution 1 ml) was collected in test tubes (preserved at 2-8 °C for the analysis) and analysed for the caffeine content with the HPLC technique.

The controls and the compositions were evaluated over three biological replicas.

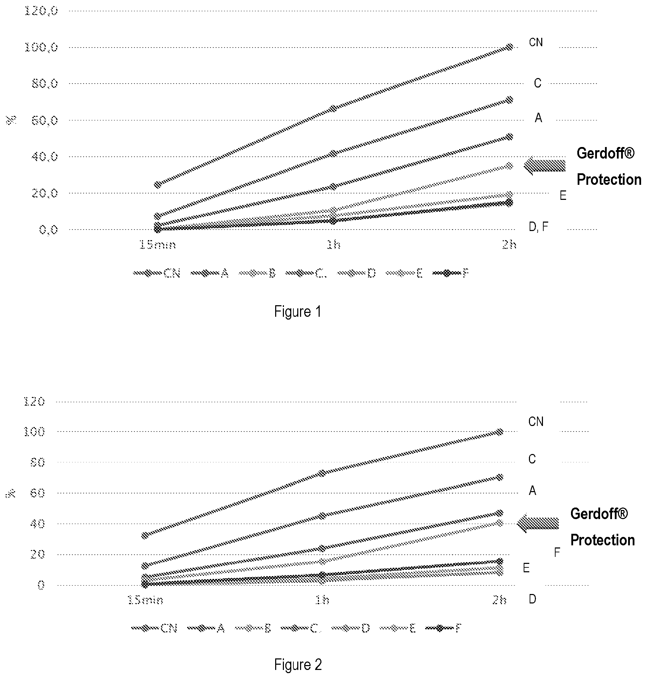

The reference product petrolatum (V), as foreseen, totally inhibits caffeine penetration (not shown in Table 1). The effectiveness of the compositions A, B and C was quantified as % of caffeine considering 100% of the dose quantified in the negative control (NC); the results are summarised in Table 3 and in Figures 1 and 2 (Samples D-F in Figure 1 and 2 are samples not according to the invention).

**Table 3**

| | **pH 7.0** | | | **pH 3.3** | | |
|---|---|---|---|---|---|---|
| **Samples** | **15min** | **1h** | **2h** | **15min** | **1h** | **2h** |
| NC | 24.5±0.8 | 41.7±0.2 | 33.7±2.5 | 32.4±0.0 | 40.4±0.5 | 27.2±0.4 |
| A | 2.3±0.1 | 21.2±0.2 | 27.2±4.5 | 5.1±0.0 | 18.8±0.5 | 23.2±1.6 |
| B | 0.3±0.0 | 10.2±0.2 | 24.4±0.9 | 2.8±0.0 | 12.3±0.0 | 25.3±3.4 |
| C | 7.3±0.0 | 34.2±0.2 | 29.5±0.1 | 12.3±0.0 | 32.8±0.1 | 25.1±0.2 |

### RESULTS

Under both of the tested conditions (pH 7 and pH 3.3) the maximum reduction in the passage of caffeine was observed with the composition according to the present invention (B). Overall, a 60% reduction was observed versus the negative control (NC). The most significant difference was observed in the first hour of treatment.

### II) Re-epithelising/reparative action

### II-i) Compositions undergoing study and Controls

The re-epithelialisation properties of:
- Comparative COMPOSITION (A)
- COMPOSITION according to the invention (B)
- Comparative ALOE: HONEY MIXTURE (C)
having the composition as illustrated in point I-i), were tested on a reconstituted human esophageal epithelium (HO2E/S/5, described in point I), in which a reproducible physical lesion was created.

The model makes it possible to investigate the processes associated with the mechanism of re-epithelialisation and evaluate the effectiveness of the products with respect to:
- undamaged tissues (negative control - NC) treated with saline solution,
- damaged tissues (positive control - INJ) treated with saline solution and petrolatum (V).

In particular, the integrity of the membrane and the inflammatory response were evaluated at the end of the treatment (24 hours) by histomorphological analysis with H&E (hematoxylin & eosin) staining.

### PROTOCOL

0.5 cm² of reconstituted human oesophageal epithelium (HOE2E/S/5) was used. The HO2E/S/5 was removed from the nutrient agar solution under a sterile laminar air flow cabin. The inserts were transferred onto a 6-well plate previously filled with maintenance medium (1 ml / well) at room temperature and incubated at 37° C, 5% CO₂, saturated humidity overnight.

The following day, for the negative control undamaged HO2E/S/5 was pre-wet with 15µL of saline solution; after 15 minutes, 30 µl of saline solution were applied for 24 hours; for the positive control and the compounds undergoing study the HO2E/S/5 was damaged and pre-wet with 15µL of saline solution for 15 minutes; then 30 µL of saline solution, V, A, B or C and controls were applied locally, directly and evenly on the damaged tissues for a treatment of 24 hours in CO₂ in the incubator at 37° C and R.H. 90%. 24 hours after treatment (T = 24 hours), the HO2E/S/5s were collected for histological analysis (H&E). The controls and the compositions were evaluated over three biological replicas.

### HISTOLOGICAL ANALYSIS (H&E staining)

The histological evaluation is useful for gaining a more thorough understanding of the type of interaction between the composition undergoing study (A, B or C) and living tissue.

At the end of the treatment (T=24 hours) the tissues are rinsed with saline solution and fixed in an adapted fixative. The tissues are sectioned (5 µm), stained with hematoxylin and eosin and analysed under a microscope.

For the positive control (damaged tissue treated with saline solution), the H&E results in the damaged area revealed the presence of necrotic cells on the surface of the epithelium, flattened migratory cells and a recovered integrity of the surface squamous layer. In the undamaged area, no significant modifications were observed versus the negative control (undamaged tissue treated with saline solution).

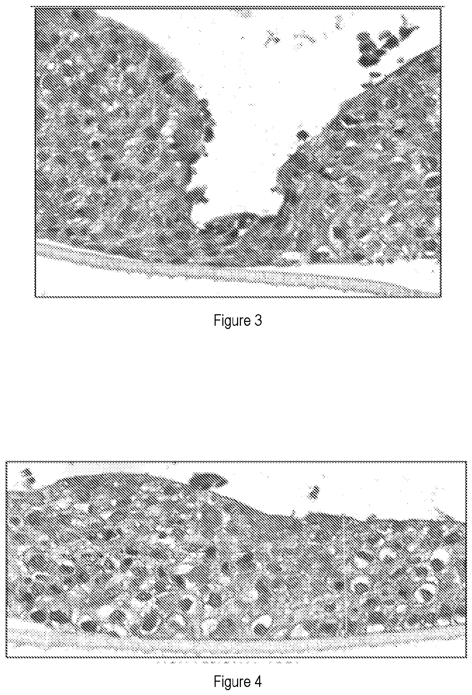

For the composition according to the invention (B), the H&E results in the damaged area (figure 3) showed a very high proliferative stimulus for repopulating the injured area. In the undamaged area (figure 4) the tissue showed a modified architecture with a greater thickness correlated with a greater production of ECM matrix. This mechanism could be associated with a healing potential in the late phase of tissue re-epithelialisation (proliferative phase following the migratory phase) and indicates a potential effectiveness of the product (B) after a long period of treatment.

Figure 3, damaged area treated with (B), shows:
- Necrotic cells: few on the surface of the epithelium.
- Flattened migratory cells: absent except in the deepest layer.
- Surface integrity (squamous): partially recovered.
- Extracellular matrix: it is not possible to arrive at any precise conclusion regarding the modifications of the ECM due to the high proliferation of the tissues and the production of ECM around the injured area.

Figure 4, undamaged area treated with (B), shows:
- Basal cells: the cells are surrounded by a modified ECM.
- Intermediate layer: reduced integrity of the cell-cell interactions and abundant ECM. Few pyknotic nuclei.
- Surface layer (squamous): unmodified.
- Extracellular matrix: overall the tissue shows a modified architecture in all layers, suggesting a high rate of proliferation in the basal layer to repair the lesion. This mechanism could be associated with a healing potential in the late phase of tissue re-epithelialisation (proliferative phase following the migratory phase).
- Thickness 97.40 µm.

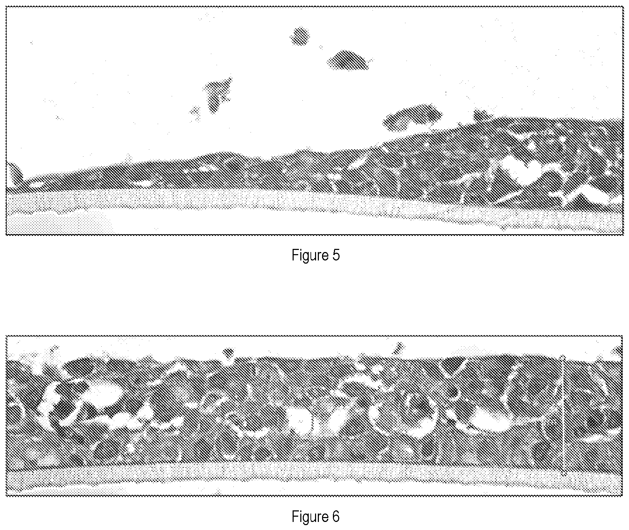

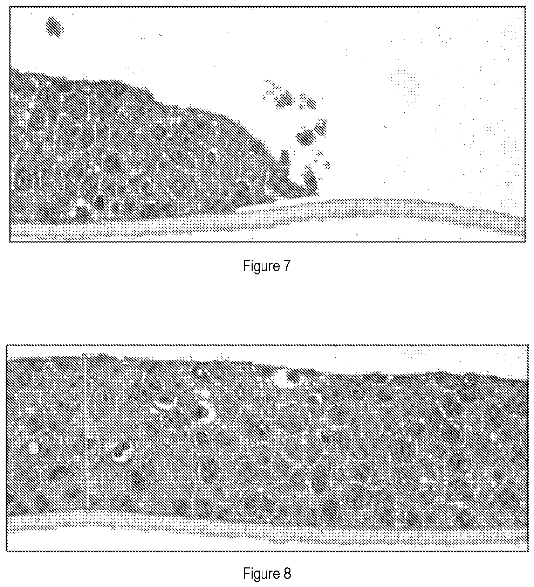

For the comparative composition (A) and ALOE:HONEY MIXTURE (C), the H&E results in the damaged area (respectively figure 5 and figure 7) confirmed that the integrity of the surface layer was lost. In the undamaged area (respectively figure 6 and figure 8) no significant modifications were observed versus the negative control in the basal layers, but a significant reduction in the cellular connections was evident, with a loss of the tissue structure in the intermediate layer.

Figure 5, damaged area treated with (A), shows:
- Necrotic cells: several as residues on the surface of the epithelium.
- Flattened migratory cells: not observed.
- Surface integrity (squamous): lost.
- Extracellular matrix: near the lesion there is a loss of tissue and extracellular matrix.

Figure 6, undamaged area treated with (A), shows:
- Basal cells: no significant modifications.
- Intermediate layer: the structure of the tissue is partially lost; significant reduction in the cell-to-cell connections.
- Surface layer (squamous): not applicable due to the reduction in thickness.
- Extracellular matrix: significant reduction in the tissue thickness and reduced matrix around the cell-cell connections.
- Thickness 65.23 µm.

### EXPERIMENTAL TRIALS (b)

The Applicant conducted *in vitro* studies to evaluate the film-forming action, on a model of human esophageal epithelium (film-forming/mucoadhesive/protective action), of a composition according to the invention compared with commercial products according to the protocol described in point (a-I-ii) of experimental trials (a).

### b-i) Compositions undergoing study and Controls

- negative control: saline solution (NC);
- positive control: white petrolatum (V);
- composition **A**: commercial product ESOXX^{®} one (liquid), comparative composition according to what is specified in Table 1;
- composition **B**: commercial product GERDOFF^{®} Protection (liquid), composition of the invention according to what is specified in Table 2;
- ALOE:HONEY MIXTURE according to what is specified in point (a-I-i);
- composition **D**: commercial product NEOBIANACID^{™} (solid), comparative composition as described below;
- composition **E**: commercial product MARIAL^{®} GEL (liquid), comparative composition as described below.

Composition **D** comprises as functional substances:
- Poliprotect^{®}: complex of polysaccharides (obtained from *Aloe vera,* Malva *sylvestris* and *Althea officinalis*) and minerals (limestone and nahcolite);
- flavonoid fraction (obtained from *Matricaria recutita* and *Glycyrrhiza glabra*).

Composition **E** comprises: E-Gastryal^{®} (hyaluronic acid, hydrolysed keratin, tara gum, xanthan gum, purified water), magnesium alginate, sucralose, potassium sorbate, sodium benzoate, ε-polylysine, flavouring, purified water.

### b-ii) Results

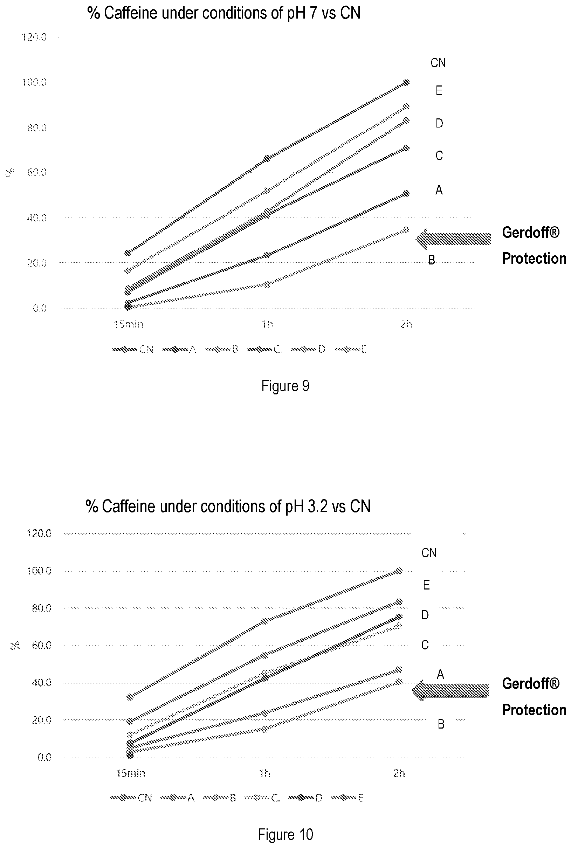

The reference product petrolatum (V), as foreseen, almost totally inhibits caffeine penetration. The effectiveness of the compositions A, B, C, D and E was quantified as % of caffeine considering the dose quantified in the negative control (NC) as 100%; the results are summarised in Table 4 and 5 and in Figure 9 and 10.

Under both conditions tested (pH 7 and pH 3.3) the maximum reduction in the passage of caffeine was observed with composition B (GERDOFF^{®} Protection), the liquid composition according to the invention. In particular, under standard pH conditions, composition B (GERDOFF^{®} Protection) showed overall the maximum effectiveness in reducing the passage of caffeine (effective passage of 34.8% after 2 hours) compared to compositions A (Esoxx^{®} one 50.8%), C (Aloe:Honey Mix, 71.0%), D (Neobianacid^{™}, 83.2%) and E (Marial^{®} gel, 89.4%), Table 4 and Figure 9. Furthermore, under acidic pH conditions, composition B (GERDOFF^{®} Protection) showed overall the maximum effectiveness in reducing the passage of caffeine (effective passage of 40.5% after 2 hours) compared to compositions A (Esoxx^{®} 47.0%), C (Aloe:Honey Mix, 70.3%), D (Neobianacid^{™}, 75.3%) and E (Marial^{®} gel, 83.5%), Table 5 and Figure 10.

**Table 4**

| | | **CAFFEINE %** | | |
|---|---|---|---|---|
| | | under conditions of pH 7 compared to negative control | | |
| | | 15min | 1h | 2h |
| CN | SALINE SOLUTION | 24.5 | 66.3 | 100.0 |
| A | ESOXX^{®} one | 2.3 | 23.6 | 50.8 |
| **B** | GERDOFF^{®} PROTECTION | 0.3 | 10.5 | **34.8** |
| C | ALOE:HONEY MIX | 7.3 | 41.5 | 71.0 |
| D | NEOBIANACID^{™} | 8.7 | 42.9 | 83.2 |
| E | MARIAL^{®} GEL | 16.7 | 52.1 | 89.4 |

**Table 5**

| | | **CAFFEINE %** | | |
|---|---|---|---|---|
| | | under conditions of pH 3.2 compared to negative control | | |
| | | 15min | 1h | 2h |
| CN | SALINE SOLUTION | 32.4 | 72.8 | 100.0 |
| A | ESOXX^{®} one | 5.1 | 23.8 | 47.0 |
| **B** | GERDOFF^{®} PROTECTION | 2.8 | 15.2 | **40.5** |
| C | ALOE:HONEY MIX | 12.3 | 45.1 | 70.3 |
| D | NEOBIANACID^{™} | 7.7 | 42.4 | 75.3 |
| E | MARIAL^{®} GEL | 19.3 | 54.9 | 83.5 |

## Claims

1. An aqueous liquid composition or hydroalcoholic liquid composition (C) comprising
(I) a mixture which comprises or, alternatively, consists of:
(a) an *Aloe vera* gel;
(b) a hyaluronic acid, or a salt thereof; and
(c) a honey, provided that said honey is not honey with a non-peroxide antibacterial activity and provided that said honey is not clover honey filtered and processed at a temperature between 100⁰ F and 140⁰ F; and, optionally,
(II) pharmaceutical or food grade additives and/or excipients, wherein said composition (C) is
- for use in a method of preventive and/or curative treatment, of laryngopharyngeal reflux (RLF) disease, or
- for use as an adjuvant in a method for the symptomatic treatment of laryngopharyngeal reflux (RLF) disease; by administration to a subject in need.

2. The composition (C) for use according to claim 1, wherein said hydroalcoholic liquid solution comprises at least one alcohol in an amount by volume comprised from 0.01% to 3%, preferably comprised from 0.05% to 2.5%, more preferably from 0.1% to 1.0%, wherein said amounts are relative to the total volume of the composition (C) and wherein said at least one alcohol is preferably ethyl alcohol.

3. The composition (C) for use according to any one of the preceding claims, wherein the hyaluronic acid has a molecular weight comprised from 400 to 900 kDa, preferably from 600 to 800 kDa.

4. The composition (C) for use according to any one of the preceding claims, wherein said mixture further comprises:
(d) a salt of chondroitin, preferably of chondroitin sulphate, more preferably a chondroitin sulphate sodium salt.

5. The composition (C) for use according to any one of the preceding claims, wherein the *Aloe vera* gel is a lyophilised *Aloe vera* inner leaf gel.

6. The composition (C) for use according to claim 5, wherein the *Aloe vera* gel is a gel obtained from *Aloe barbadensis* Miller.

7. The composition (C) for use according to any one of the preceding claims, wherein the components (a), (b), (c) and, if present, (d) are present in the amounts:
(a) is in an amount comprised from 0.01% to 5%, preferably comprised from 0.1% to 0.5%
(b) is in an amount comprised from 0.01% to 5%, preferably comprised from 0.1% to 0.5%
(c) is in an amount comprised from 5% to 50%, preferably comprised from 10% to 40%; and
(d), if present, is in an amount comprised from 0.1% to 10%, preferably comprised from 1% to 4%,
wherein all the % amounts are by weight relative to the total weight of (C).

8. The composition (C) for use according to any one of the preceding claims, wherein said composition (C) comprises per 100 ml of (C):
- (a) in an amount by weight comprised from 100 mg to 500 mg, preferably comprised from 150 mg to 300 mg;
- (b) in an amount by weight comprised from 100 mg to 500 mg, preferably comprised from 100 mg to 300 mg;
- (c) in an amount by weight comprised from 10 g to 50 g, preferably comprised from 20 g to 30 g; optionally
- (d) in an amount by weight comprised from 1 g to 5 g, preferably comprised from 2 g to 3 g.

9. An hydroalcoholic liquid composition (C) comprising
(I) a mixture which comprises or, alternatively, consists of:
(a) an *Aloe vera* gel;
(b) a hyaluronic acid, or a salt thereof; and
(c) a honey, provided that said honey is not honey with a non-peroxide antibacterial activity and provided that said honey is not clover honey filtered and processed at a temperature between 100⁰ F and 140⁰ F; and, optionally,
(II) pharmaceutical or food grade additives and/or excipients.

10. The composition (C) according to claim 9, wherein said hydroalcoholic liquid solution comprises at least one alcohol in an amount by volume comprised from 0.01% to 3%, preferably comprised from 0.05% to 2.5%, more preferably from 0.1% to 1.0%, wherein said amounts are relative to the total volume of the composition (C).

11. The composition (C) according to claim 10, wherein said at least one alcohol is ethyl alcohol.

## Patentansprüche

1. Wässrige flüssige Zusammensetzung oder hydroalkoholische flüssige Zusammensetzung (C), umfassend
(I) ein Gemisch, das Folgendes umfasst oder alternativ dazu aus Folgendem besteht:
(a) ein *Aloe* Vera-Gel;
(b) eine Hyaluronsäure oder ein Salz davon; und
(c) einen Honig, vorausgesetzt, dass der Honig kein Honig mit einer nicht peroxidischen antibakteriellen Aktivität ist und vorausgesetzt, dass der Honig kein Kleehonig ist, der bei einer Temperatur zwischen 100° F und 140° F gefiltert und verarbeitet wird; und gegebenenfalls
(II) pharmazeutische oder lebensmittelechte Zusatzstoffe und/oder Hilfsstoffe, wobei die Zusammensetzung (C) ist
- zur Verwendung in einem Verfahren zur vorbeugenden und/oder heilenden Behandlung von der Krankheit von laryngopharyngealem Reflux (RLF);
oder
- zur Verwendung als Adjuvans in einem Verfahren zur symptomatischen Behandlung von der Krankheit von laryngopharyngealem Reflux (RLF);
durch Verabreichung an einen bedürftigen Probanden.

2. Zusammensetzung (C) zur Verwendung nach Anspruch 1, wobei die hydroalkoholische flüssige Lösung mindestens einen Alkohol in einer Volumenmenge von 0,01 % bis 3 %, vorzugsweise von 0,05 % bis 2,5 %, bevorzugter von 0,1 % bis 1,0 % umfasst, wobei die Mengen auf das Gesamtvolumen der Zusammensetzung (C) bezogen sind und wobei der mindestens eine Alkohol vorzugsweise Ethylalkohol ist.

3. Zusammensetzung (C) zur Verwendung nach einem der vorhergehenden Ansprüche, wobei die Hyaluronsäure ein Molekulargewicht von 400 bis 900 kDa, vorzugsweise von 600 bis 800 kDa, aufweist.

4. Zusammensetzung (C) zur Verwendung nach einem der vorhergehenden Ansprüche, wobei das Gemisch ferner umfasst:
(d) ein Salz von Chondroitin, vorzugsweise von Chondroitinsulfat, bevorzugter ein Chondroitinsulfat-Natriumsalz.

5. Zusammensetzung (C) zur Verwendung nach einem der vorhergehenden Ansprüche, wobei das *Aloe* Vera-Gel ein lyophilisiertes inneres Blattgel von *Aloe Vera* ist.

6. Zusammensetzung (C) zur Verwendung nach Anspruch 5, wobei das *Aloe* Vera-Gel ein Gel ist, das aus *Aloe barbadensis* Miller erhalten wird.

7. Zusammensetzung (C) zur Verwendung nach einem der vorhergehenden Ansprüche, wobei die Komponenten (a), (b), (c) und, falls vorhanden, (d) in den folgenden Mengen vorhanden sind:
(a) in einer Menge von 0,01 % bis 5 %, vorzugsweise 0,1 % bis 0,5 %, ist,
(b) in einer Menge von 0,01 % bis 5 %, vorzugsweise von 0,1 % bis 0,5 %, ist,
(c) in einer Menge von 5 % bis 50 %, vorzugsweise von 10 % bis 40 %, ist; und
(d) falls vorhanden, in einer Menge von 0,1 % bis 10 %, vorzugsweise von 1 % bis 4 %, ist,
wobei alle %-Mengen nach Gewicht, bezogen auf das Gesamtgewicht von (C) sind.

8. Zusammensetzung (C) zur Verwendung nach einem der vorhergehenden Ansprüche, wobei die Zusammensetzung (C) pro 100 ml von (C) umfasst:
- (a) in einer Gewichtsmenge von 100 mg bis 500 mg, vorzugsweise von 150 mg bis 300 mg;
- (b) in einer Gewichtsmenge von 100 mg bis 500 mg, vorzugsweise von 100 mg bis 300 mg;
- (c) in einer Gewichtsmenge von 10 g bis 50 g, vorzugsweise von 20 g bis 30 g;
gegebenenfalls
- (d) in einer Gewichtsmenge von 1 g bis 5 g, vorzugsweise von 2 g bis 3 g.

9. Hydroalkoholische flüssige Zusammensetzung (C), umfassend
(I) ein Gemisch, das Folgendes umfasst oder alternativ dazu aus Folgendem besteht:
(a) ein *Aloe* Vera-Gel;
(b) eine Hyaluronsäure oder ein Salz davon; und
(c) einen Honig, vorausgesetzt, dass der Honig kein Honig mit einer nicht peroxidischen antibakteriellen Aktivität ist und vorausgesetzt, dass der Honig kein Kleehonig ist, der bei einer Temperatur zwischen 100° F und 140° F gefiltert und verarbeitet wird; und gegebenenfalls
(II) pharmazeutische oder lebensmittelechte Zusatzstoffe und/oder Hilfsstoffe.

10. Zusammensetzung (C) nach Anspruch 9, wobei die hydroalkoholische flüssige Lösung mindestens einen Alkohol in einer Volumenmenge von 0,01 % bis 3 %, vorzugsweise von 0,05 % bis 2,5 %, bevorzugter von 0,1 % bis 1,0 % umfasst, wobei die Mengen auf das Gesamtvolumen der Zusammensetzung (C) bezogen sind.

11. Zusammensetzung (C) nach Anspruch 10, wobei der mindestens eine Alkohol Ethylalkohol ist.

## Revendications

1. Composition liquide aqueuse ou composition liquide hydroalcoolique (C), comprenant
(I) un mélange qui comprend ou, alternativement, est constitué :
(a) d'un gel d'*aloé vera* ;
(b) d'un acide hyaluronique ou un de ses sels ; et
(c) d'un miel, à condition que ce miel ne soit pas un miel ayant une activité antibactérienne non peroxyde et qu'il ne s'agisse pas d'un miel de trèfle filtré et traité à une température comprise entre 100° F et 140° F ; et, éventuellement,
(II) d'additifs et/ou excipients de qualité pharmaceutique ou alimentaire, dans laquelle ladite composition (C) est
- destinée à être utilisée dans une méthode de traitement préventif et/ou curatif de la maladie du reflux laryngopharyngien (RLF),
ou
- destinée à être utilisée comme un adjuvant dans une méthode de traitement symptomatique de la maladie du reflux laryngopharyngien (RLF) ;
par administration à un sujet qui en a besoin.

2. Composition (C) à utiliser selon la revendication 1, dans laquelle ladite solution liquide hydroalcoolique comprend au moins un alcool dans une quantité en volume comprise entre 0,01 et 3 %, de préférence entre 0,05 et 2,5 %, plus préférablement entre 0,1 et 1,0 %, dans laquelle lesdites quantités étant relatives au volume total de la composition (C) et ledit au moins un alcool étant de préférence de l'alcool éthylique.

3. Composition (C) à utiliser selon l'une quelconque des revendications précédentes, dans laquelle l'acide hyaluronique a un poids moléculaire compris entre 400 et 900 kDa, de préférence entre 600 et 800 kDa.

4. Composition (C) à utiliser selon l'une quelconque des revendications précédentes, dans laquelle ledit mélange comprend de plus :
(d) un sel de chondroïtine, de préférence du sulfate de chondroïtine, plus préférablement un sel de sodium de sulfate de chondroïtine.

5. Composition (C) à utiliser selon l'une quelconque des revendications précédentes, dans laquelle le gel d'aloé *vera* est un gel de feuille intérieure d'aloé *vera* lyophilisée.

6. Composition (C) à utiliser selon la revendication 5, dans laquelle le gel d'aloé *vera* est un gel obtenu à partir d'Aloe *barbadensis Miller.*

7. Composition (C) à utiliser selon l'une quelconque des revendications précédentes, dans laquelle les composants (a), (b), (c) et, le cas échéant, (d) sont présents dans les quantités suivantes :
(a) dans une quantité comprise entre 0,01 et 5 %, de préférence comprise entre 0,1 et 0,5 %,
(b) dans une quantité comprise entre 0,01 et 5 %, de préférence comprise entre 0,1 et 0,5 %,
(c) dans une quantité comprise entre 5 et 50 %, de préférence entre 10 et 40 % ; et
(d) s'il est présent, dans une quantité comprise entre 0,1 et 10 %, de préférence entre 1 et 4 %,
dans laquelle tous les pourcentages sont exprimés en poids par rapport au poids total de (C).

8. Composition (C) à utiliser selon l'une quelconque des revendications précédentes, dans laquelle ladite composition (C) comprend pour 100 ml de (C) :
- (a) dans une quantité en poids comprise entre 100 et 500 mg, de préférence comprise entre 150 et 300 mg ;
- (b) dans une quantité en poids comprise entre 100 et 500 mg, de préférence entre 100 et 300 mg ;
- c) dans une quantité en poids comprise entre 10 et 50 g, de préférence entre 20 et 30 g ;
éventuellement
- d) dans une quantité en poids comprise entre 1 et 5 g, de préférence entre 2 et 3 g.

9. Composition liquide hydroalcoolique (C), comprenant
(I) un mélange qui comprend ou, alternativement, est constitué :
(a) d'un gel d'*aloé vera* ;
(b) d'un acide hyaluronique ou un de ses sels ; et
(c) d'un miel, à condition que ce miel ne soit pas un miel ayant une activité antibactérienne non peroxyde et qu'il ne s'agisse pas d'un miel de trèfle filtré et traité à une température comprise entre 100° F et 140° F ; et, éventuellement,
(II) d'additifs et/ou excipients de qualité pharmaceutique ou alimentaire.

10. Composition (C) selon la revendication 9, dans laquelle ladite solution liquide hydroalcoolique comprend au moins un alcool dans une quantité en volume comprise entre 0,01 et 3 %, de préférence entre 0,05 et 2,5 %, plus préférablement entre 0,1 et 1,0 %, lesdites quantités étant relatives au volume total de la composition (C).

11. Composition (C) selon la revendication 10, dans laquelle ledit au moins un alcool est de l'alcool éthylique.
